(19) Europäisches Patentamt
European Patent Office
Office européen des brevets

(11) Publication number : **0 378 358 B1**

# (12) EUROPEAN PATENT SPECIFICATION

(45) Date of publication of patent specification : **03.11.93 Bulletin 93/44**

(51) Int. Cl.[5] : **C07D 405/04,** C07D 405/14, C07D 409/04, C07D 409/14, C07D 417/04, C07D 417/14, A61K 31/40, A61K 31/425

(21) Application number : **90300229.3**

(22) Date of filing : **09.01.90**

## (54) Anti-inflammmatory 1-heteroaryl-3-acyl-2-oxindoles.

(30) Priority : **10.01.89 PCT/US89/00079**

(43) Date of publication of application : **18.07.90 Bulletin 90/29**

(45) Publication of the grant of the patent : **03.11.93 Bulletin 93/44**

(84) Designated Contracting States : **AT BE CH DE DK ES FR GB GR IT LI LU NL SE**

(56) References cited :
**EP-B- 0 153 818**
**US-A- 4 556 672**
**US-A- 4 569 942**
**US-A- 4 690 943**
**US-A- 4 721 712**

(73) Proprietor : **PFIZER INC.**
**235 East 42nd Street**
**New York, N.Y. 10017 (US)**

(72) Inventor : **Marfat, Anthony**
**160 Lantern Hill Road**
**Mystic, Connecticut (US)**

(74) Representative : **Wood, David John et al**
**PFIZER LIMITED, Ramsgate Road**
**Sandwich, Kent CT13 9NJ (GB)**

EP 0 378 358 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to new and useful non-steroidal anti-inflammatory agents. More particularly, it relates to certain 1-heteroaryl-3-acyl-2-oxindoles which are potent inhibitors of cyclooxygenase (CO) and lipoxygenase (LO) and which are of value as anti-inflammatory agents in mammals.

U.S. patent 4,556,672, issued December 3, 1985, describes a series of 3-acyl-2-oxindole-1-carboxamides in which the acyl group, -C(=O)R, is derived from a wide variety of organic carboxylic acids, including those wherein R is phenyl, substituted phenyl, thienyl, furyl or other heterocyclyl groups. The compounds are useful as analgesics and anti-inflammatory agents. Related compounds in which the 1-carboxamide group is substituted by alkyl, phenyl, substituted phenyl, furyl or thienyl are described in U.S. patent 4,569,942, issued February 11, 1986, as having the same utility. Also disclosed in each of said patents are 3-acyl-2-oxindoles wherein the acyl group is as defined above.

Analgesic and anti-inflammatory 1,3-diacyl-2-oxindoles wherein the 1-acyl group is an alkanoyl group and the 3-acyl group is as disclosed in the above-mentioned patents are described in U.S. patent 4,690,943, issued September 1, 1987.

It has been found that certain 1-heteroaryl-3-acyl-2-oxindoles of formula (I)

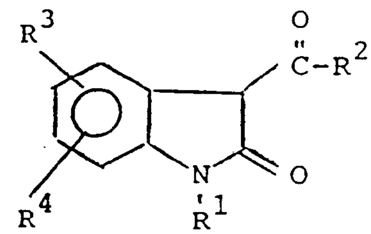

(I)

or a pharmaceutically acceptable base salt thereof wherein

$R^1$ is thienyl, furyl or 2-thiazolyl;

$R^2$ is thienyl, furyl or

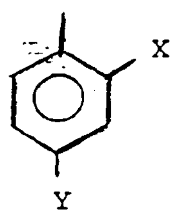

wherein X is hydrogen, fluoro, chloro, alkyl having 1 to 4 carbon atoms, alkoxy having 1 to 4 carbon atoms, alkylthio having 1 to 4 carbon atoms, nitro or trifluoromethyl;

Y is hydrogen, fluoro or chloro;

and each of $R^3$ and $R^4$, which may be alike or different, is hydrogen, fluoro, chloro, bromo, alkyl having 1 to 4 carbon atoms, alkoxy having 1 to 4 carbon atoms, alkylthio having 1 to 4 carbon atoms, nitro or trifluoromethyl are potent inhibitors of CO and LO enzymes, and are of value as anti-inflammatory agents for treating an inflammatory disease in a mammal. They are especially useful for treatment of rheumatoid arthritis in mammals.

A favored group of compounds are those wherein $R^2$ is phenyl or substituted phenyl. Within this group of compounds those wherein one or both of X and Y are hydrogen, fluoro or chloro are preferred compounds. Especially preferred in this group are those compounds wherein each of X and Y is hydrogen and $R^1$ is a 3-heteroaryl group.

Another favored group of compounds embraces those wherein $R^2$ is thienyl or furyl. Preferred within this group are those wherein $R^2$ is 2-thienyl or 2-furyl and $R^1$ is a 3-heteroaryl group.

Further preferred is a group of compounds wherein $R^1$ is 2-thienyl or 3-thienyl and $R^2$ is

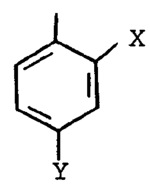

preferably either where X is H, F, Cl, -S($C_1$-$C_4$ alkyl) or $CF_3$, Y is H, F or Cl and each of $R^3$ and $R^4$ is hydrogen, or where each of X and Y is H, F or Cl and each of $R^3$ and $R^4$ is hydrogen.

Yet further preferred is a group of compounds wherein $R^1$ is 3-furyl and $R^2$ is

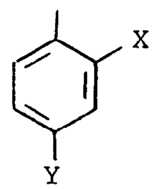

preferably where each of X and Y is H, F or Cl and each of $R^3$ and $R^4$ is H.

Included within this invention are pharmaceutical compositions, useful as anti-inflammatory agents in a mammal, comprising a pharmaceutically acceptable carrier and an inflammatory disease treating amount of a compound of formula (I).

The compounds of this invention are readily prepared from appropriate reactants by the following process:

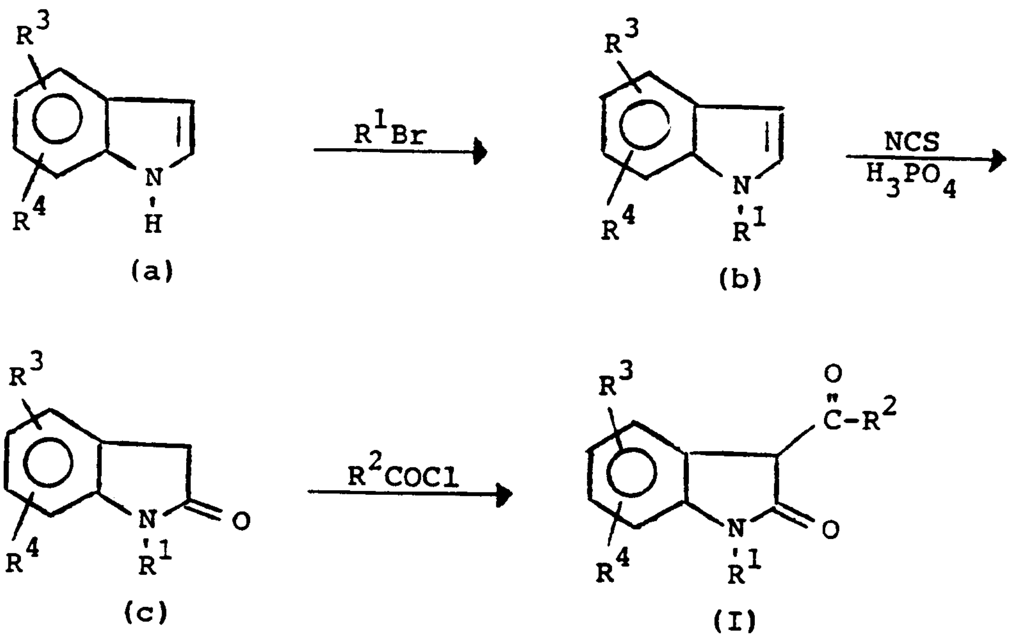


(a) (b) (c) (I)

The first step of the overall process is carried out by reacting indole (or an appropriately substituted indole) with the desired $R^1Br$ in a suitable solvent in the presence of a base and a catalytic amount of cuprous bromide. As solvent, N-methyl-2-pyrrolidinone serves especially well. Other aprotic solvents such as dimethylsulfoxide, dimethylformamide or diethylformamide can be used. The reaction is conducted at an elevated temperature, e.g. from 50°C. to 200°C., desirably under a nitrogen atmosphere. The choice of base is not critical, but can be any of a variety of inorganic and organic bases. Representative of such bases are alkali metal hydrides, carbonates or hydroxides; triethylamine, pyridine, N-methylmorpholine, 4-(N,N-dimethylamino)pyridine and N-methylpiperidine.

The above-named ingredients are all generally placed in a reaction vessel, stirred and heated to the desired temperature until reaction is complete. Although the indole, $R^1Br$, and base reactants can be used in stoichiometric proportions, it is preferred to use 5-10% excess of each of $R^1Br$ and base in order to expedite the

reaction. A greater excess of $R^1Br$, e.g., up to 100%, can be used but is generally avoided for economic reasons. The cuprous bromide is employed at a level of from 0.01 to 0.03 moles per mole of $R^1Br$ reactant. The product is recovered by known methods. The required indole reactants are known compounds or are conveniently prepared according to known procedures.

The second step, conversion of the 1-substituted indole (b) to the corresponding oxindole (c) is accomplished by reacting indole (b) with N-chlorosuccinimide (NCS) in a reaction-inert solvent at ambient temperature. A 5-10% excess of NCS is generally used. Reaction periods of from 1 to 5 hours, depending upon the indole (a) reactant normally lead to complete reaction. The reaction-inert solvent (i.e., one which does not react with reactants or products) can be any of a variety of solvents, such as diethyl ether, dioxane, tetrahydrofuran, aromatic hydrocarbons, (benzene, toluene, xylene), chloroform, acetonitrile and mixtures thereof.

Upon completion of the NCS reaction, the reaction is concentrated under reduced pressure and the 3-chloro indole derivative taken up in glacial acetic acid and heated to from 50°-80°C. Phosphoric acid (85%) is then added to the reaction which is refluxed for 1-24 hours, cooled and poured into ice-water. The aqueous mixture is adjusted to pH 11-12 and then extracted with ethyl acetate to recover the oxindole. Work-up of the extract and purification of the oxindole product is by standard methods.

Introduction of the acyl moiety at the 3-position of oxindole (c) is conveniently accomplished by reacting (c) with an activated derivative of the appropriate carboxylic acid of formula $R^2$-COOH in a lower alkanol solvent (e.g. ethanol) in the presence of an alkali metal salt of the lower alkanol solvent (e.g. sodium ethoxide) according to standard procedures. Typical derivatives of the acids include acid halides, e.g., acid chlorides; symmetrical acid anhydrides, $R^2$-C(=O)-O-C(=O)-$R^2$; mixed acid anhydrides with a hindered low-molecular weight carboxylic acid, $R^2$-C(=O)-O-C(=O)-$R^5$, where $R^5$ is a bulky lower-alkyl group such as t-butyl; and mixed carboxylic-carbonic anhydrides, $R^2$-C(=O)-O-C(=O)-O$R^6$, wherein $R^6$ is a lower-alkyl group. Usually, a small excess of the derivative of the acid of formula $R^2$-C(=O)-OH is used, and the alkoxide salt is usually present in an amount from one to two molar equivalents, based on said derivative of the acid of formula $R^2$-C(=O)-OH. The reaction between the derivative of the acid of the formula $R^2$-C(=O)-OH and the compound of formula (c) is usually started at 0° to 25° C., but it is then usual to heat the reaction mixture at a temperature in the range from 50° to 130° C., and preferably at about 80° C., to complete the reaction. Under these circumstances, reaction times of a few hours, e.g., two hours, up to a few days, e.g., two days, are commonly used. The reaction mixture is then cooled, diluted with an excess of water and acidified. The product of formula (I) can then be recovered by filtration or by the standard procedure of solvent extraction.

A common characteristic of the oxindole carboxamides of this invention is their acidic nature. Therefore, included in this invention are the pharmaceutically acceptable salts of the compounds of formula (I). The preferred cations of said salts include the ammonium, sodium and potassium ions. The pharmaceutically acceptable salts of the compounds described herein are prepared by conventional procedures, as for example, by adding the acid to an aqueous solution containing an equivalent amount of the pharmaceutically acceptable base, i.e., a base containing one of the above preferred cations, followed by concentration of the resultant mixture to obtain the desired product. The bases can be selected from hydroxides, oxides or carbonates.

Also considered part of the present invention are prodrugs of the herein described compounds of formula (I). These prodrugs, which have fewer gastrointestinal side effects, break down in situ to the parent compound. Representative prodrugs of formula (I) compounds are enol esters and ethers thereof of formula (II)

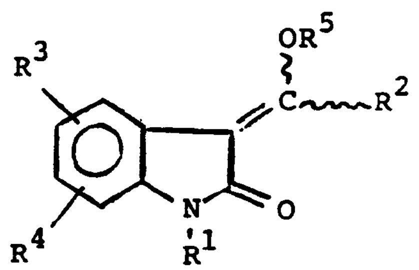

(II)

wherein each of $R^1$, $R^2$, $R^3$ and $R^4$ is as defined above; and $R^5$ is alkanoyl, cycloalkylcarbonyl, phenylalkanoyl, chlorobenzoyl, methoxybenzyl, phenyl, thenoyl, omegaalkoxycarbonylalkanoyl, alkoxycarbonyl, phenoxycarbonyl, 1-alkoxyalkyl, 1-alkoxycarbonyloxyalkyl, alkyl, alkylsulfonyl, methylphenylsulfonyl or dialkylphosphonate.

In formula (II), the wavy lines on the carbon atom of the exocyclic double bond at the 3-position are intended to represent the syn-, the anti- and mixtures of the syn- and anti- forms of formula (II) compounds.

Formula (II) compounds are prepared by treating the appropriate 3-acyl 2-oxindole of formula (I) and an

equimolar amount of triethylamine in a reaction-inert solvent (e.g., chloroform) with a slight excess of the requisite acid chloride, chloroformate, oxonium salt or alkylating agent at 0° C. The reaction is allowed to warm to room temperature and, after 2-3 hours, the product is recovered by known procedures.

A second procedure for preparation of formula (II) compounds consists of contacting, in an anhydrous reaction-inert solvent such as acetone, the appropriate 3-acyl-2-oxindole of formula (I), a three-fold molar excess of the requisite alpha-chloroalkylcarbonate, a five-fold molar excess of sodium iodide and a two-fold molar excess of anhydrous potassium carbonate and heating said reaction mixture at reflux for 16 hours.

The reaction mixture is diluted with water and the product extracted with a water-immiscible solvent, such as diethyl ether or chloroform. Concentration of the solvent containing the product provides the crude material, which can be purified by recrystallization and/or chromatography.

As previously indicated, the oxindole carboxamides of the present invention and their pharmaceutically acceptable salts are useful anti-inflammatory agents in mammals. These compounds are of value in alleviating swelling and inflammation which are symptomatic of rheumatoid arthritis and related disorders which are responsive to treatment with anti-inflammatory agents. Either as individual therapeutic agents or as mixtures of therapeutic agents, they may be administered alone, but are generally administered with a pharmaceutical carrier selected on the basis of the chosen route of administration and standard pharmaceutical practice. For example, they may be administered orally in the form of tablets or capsules containing such excipients as starch, milk sugar or certain types of clay, etc. They may be administered orally in the form of elixirs or oral suspensions with the active ingredients combined with emulsifying and/or suspending agents. They may be injected parenterally, and for this use they, or appropriate derivatives, may be prepared in the form of sterile aqueous solutions. Such aqueous solutions should be suitably buffered, if necessary, and should contain other solutes such as saline or glucose to render them isotonic. The weight-ratio of the pharmaceutically-acceptable carrier to compound can be from 1:4 to 20:1.

The dosage required to reduce inflammation or swelling in arthritic subjects would be determined by the nature and extent of the symptoms. Generally, small doses will be required initially, with a gradual increase in the dose until the optimum level is determined. It will generally be found that when the composition is administered orally, larger amounts of the active ingredient will be required to produce the same level as produced by a smaller quantity administered parenterally. In general, from about 10 to about 300 mg of active ingredient per kilogram of body weight, administered orally in single or multiple dose units, will effectively reduce inflammation and swelling. Parenteral administration requires doses of from about 5 to about 200 mg of active ingredient to achieve the same end point.

A standard procedure for detecting and comparing anti-inflammatory activity of compounds is the carrageenin rat foot edema test, which is described by C. A. Winter et al., Proc. Soc. Exp. Biol. vol III, page 544 (1962).

In addition to being useful as anti-inflammatory agents, the compounds of the present invention can be used in the treatment of asthma, bronchitis and psoriasis; they can also be used as analgesic agents.

The following examples are provided solely for the purpose of further illustration. Nuclear magnetic resonance spectra (NMR) were measured at 60 MHz for solutions in deuterochloroform ($CDCl_3$) and peak positions are expressed in parts per million (ppm) downfield from tetramethylsilane or sodium 2,2-dimethyl-2-silapentane-5-sulfonate. The following abbreviations for peak shapes are used: s, singlet; d, doublet; t, triplet; q, quartet; m, multiplet; b, broad.

## EXAMPLE 1

### 1-(2-Thiazolyl)-3-(2-furoyl)-2-oxindole

To a solution of 1-(2-thiazolyl)-2-oxindole (0.6 g, 2.7 mM) in dimethylformamide (8 ml) at 0° C. was added 4-dimethylaminopyridine (0.847 g, 2.2 eq.) and the mixture stirred for 5 minutes. Then, a solution of 2-furoyl chloride (0.31 ml, 3.04 mM, 1.1 eq.) in dimethylformamide (2 ml) was added dropwise and the mixture stirred for 15 minutes at 0° C. and then at room temperature for 2 hours. The reaction was poured onto about 100 ml of ice/2N HCl, and the resulting yellow precipitate recovered by filtration, washed with water and air-dried. Recrystallization from toluene-hexane (1:1) afforded 0.585 g (64%) of title product as yellow needles; m.p. 156-157° C.

In like manner the following compounds were prepared from appropriate reactants.

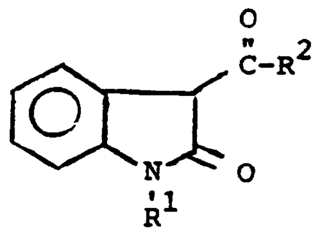

| $R^1$ | $R^2$ | (m.p. °C.) |
|---|---|---|
| 2-thiazolyl | 2-thienyl | 168-9 |
| 3-thienyl | 2-furyl | 132-3 |
| 3-thienyl | 2-thienyl | 130-2 (dec.) |

EXAMPLE 2

Following the procedure of Example 1, the compounds listed below are prepared from appropriate reactants:

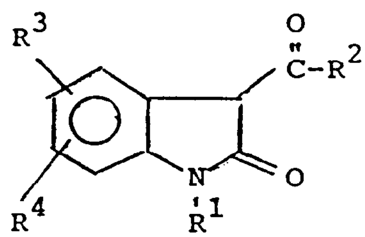

| $R^3$ | $R^4$ | $R^1$ | $R^2$ |
|---|---|---|---|
| H | H | 2-thiazolyl | $C_6H_5$ |
| H | H | 2-thiazolyl | $4-FC_6H_4$ |
| H | H | 2-thiazolyl | $4-ClC_6H_4$ |
| H | H | 2-thiazolyl | $2,4-F_2C_6H_3$ |
| H | H | 2-thiazolyl | 3-thienyl |
| H | H | 2-thiazolyl | $2-CF_3C_6H_4$ |
| 5-Cl | H | 2-thiazolyl | 2-thienyl |
| 5-Cl | H | 2-thiazolyl | 3-furyl |
| 6-F | H | 2-thiazolyl | $C_6H_5$ |
| H | 7-F | 2-thiazolyl | $2,4-Cl_2C_6H_3$ |
| 5-F | 6-F | 2-thiazolyl | $4-FC_6H_4$ |
| H | $7-C_2H_5$ | 2-thiazolyl | 3-thienyl |

| $R^3$ | $R^4$ | $R^1$ | $R^2$ |
|---|---|---|---|
| 4-$OCH_3$ | H | 2-thiazolyl | 2-furyl |
| H | 6-$CF_3$ | 2-thiazolyl | 4-$ClC_6H_4$ |
| H | H | 3-thienyl | $C_6H_5$ |
| H | H | 3-thienyl | 4-$ClC_6H_4$ |
| H | H | 3-thienyl | 4-$FC_6H_4$ |
| H | H | 3-thienyl | 2,4-$Cl_2C_6H_3$ |
| H | H | 3-thienyl | 2-$CF_3C_6H_4$ |
| 5-F | H | 3-thienyl | 3-thienyl |
| 5-F | 6-F | 3-thienyl | 3-thienyl |
| 5-$NO_2$ | H | 3-thienyl | 2-furyl |
| 4-$OCH_3$ | 6-$OCH_3$ | 3-thienyl | $C_6H_5$ |
| 5-$OCH_3$ | 6-Cl | 3-thienyl | 4-$FC_6H_4$ |
| 5-$CF_3$ | H | 3-thienyl | 2-furyl |
| 5-Br | H | 3-thienyl | $C_6H_5$ |
| 6-i-$C_3H_7$ | H | 3-thienyl | 4-$ClC_6H_4$ |
| H | H | 2-thienyl | 3-thienyl |
| 4-$SCH_3$ | H | 2-thienyl | 3-furyl |
| 5-Cl | 6-Cl | 2-thienyl | $C_6H_5$ |
| 6-F | H | 2-thienyl | $C_6H_5$ |
| 5-$OCH_3$ | 6-F | 2-thienyl | $C_6H_5$ |
| 4-$C_2H_5$ | H | 2-thienyl | 3-thienyl |
| 5-Cl | 6-Cl | 2-thienyl | 2-thienyl |

| $R^3$ | $R^4$ | $R^1$ | $R^2$ |
|---|---|---|---|
| 5-Br | H | 2-thienyl | $C_6H_5$ |
| 4-$OCH_3$ | 7-$OCH_3$ | 2-thienyl | 2,4-$F_2C_6H_3$ |
| 5-$CF_3$ | H | 2-thienyl | 2-thienyl |
| H | H | 3-furyl | 3-thienyl |
| H | H | 3-furyl | 3-furyl |
| H | H | 3-furyl | $C_6H_5$ |
| H | H | 3-furyl | 4-$ClC_6H_4$ |
| 5-F | 6-F | 3-furyl | $C_6H_5$ |
| H | 6-F | 3-furyl | $C_6H_5$ |
| 5-Cl | H | 3-furyl | $C_6H_5$ |
| 5-$CH_3$ | H | 3-furyl | 4-$FC_6H_4$ |
| 5-$OCH_3$ | 6-Cl | 3-furyl | 4-$ClC_6H_4$ |
| 5-$CH_3$ | 6-$CH_3$ | 3-furyl | 4-$FC_6H_4$ |
| 5-$CF_3$ | H | 3-furyl | 2,4-$F_2C_6H_3$ |
| 5-$NO_2$ | H | 3-furyl | 2-$ClC_6H_4$ |
| H | H | 2-furyl | 2-thienyl |
| H | H | 2-furyl | 3-furyl |
| H | H | 2-furyl | $C_6H_5$ |
| H | H | 2-furyl | 2-$FC_6H_4$ |
| H | H | 2-furyl | 4-$ClC_6H_4$ |

| $R^3$ | $R^4$ | $R^1$ | $R^2$ |
|---|---|---|---|
| 5-F | 6-F | 2-furyl | $C_6H_5$ |
| H | 6-Cl | 2-furyl | $4\text{-}ClC_6H_4$ |
| $5\text{-}OCH_3$ | 6-F | 2-furyl | $4\text{-}FC_6H_4$ |
| H | $7\text{-}i\text{-}C_4H_9$ | 2-furyl | $2,4\text{-}F_2C_6H_3$ |
| $4\text{-}NO_2$ | 7-Cl | 2-furyl | $C_6H_5$ |
| 5-Br | $6\text{-}CH_3$ | 2-furyl | $4\text{-}ClC_6H_4$ |

PREPARATION A

1-(3-Thienyl)indole

A mixture of indole (16 g, 0.136 M), 3-bromothiophene (24.75 g, 0.146 M), potassium carbonate (20.1 g, 0.146 M) and copper bromide (0.84 g, 0.003 M) in 160 ml of N-methyl-2-pyrrolidinone was stirred and heated to 180° C. (under nitrogen atmosphere) for 42 hours. After this time, the reaction mixture was poured onto 800 ml of water and extracted with ethyl acetate (2 x 350 ml). The combined ethyl acetate extracts were washed with water, brine, dried over magnesium sulfate, filtered and concentrated in vacuo leaving a dark brown-black oil. The crude reaction product was chromatographed on a silica gel column with hexane/$CH_2Cl_2$ 3:1 as eluant. Yield = 9.14 g of yellow liquid which was homogeneous by thin layer chromatography. MS: $M^+$ = 199.

| | |
|---|---|
| Analysis calculated for $C_{12}H_9NS$: | |
| | C, 72.32; H, 4.55; N, 7.23%. |
| Found: | C, 72.06; H, 4.71; N, 7.47%. |

In like manner the following 1-(heteroaryl)indoles are prepared from the appropriate bromoheteroaryl reactant:

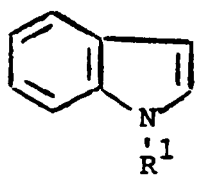

| $R^1$ | MS: $M^+$ |
|---|---|
| 2-thienyl | 199 |

| $R^1$ | MS: $M^+$ |
|---|---|
| 3-furyl[a] | 183 (100) |
| 2-thiazolyl[b] | 200 (100) |

[a] $R_f(CH_2Cl_2) = 0.67$

[b] a 100% excess of 2-thiazolyl bromide was used. $R_f$ of product in $CH_2Cl_2 = 0.45$.

PREPARATION B

1-(3-Thienyl)oxindole

To a solution of 1-(3-thienyl)indole (9.14 g, 0.0459 M) in 350 ml of dry methylene chloride under a nitrogen atmosphere was added 6.44 g (0.0482 M) of N-chlorosuccinimide (NCS) at room temperature. The reaction was stirred at room temperature for 2 hours, then concentrated in vacuo. The resulting foamy residue was immediately dissolved in 190 ml of glacial acetic acid. The resulting mixture was heated to 70° C. followed by the addition of 49.5 ml of 85% $H_3PO_4$ and heating of the reaction mixture to reflux for one hour. The mixture was then cooled to room temperature, poured onto ice water, basified to pH 11-12 with $Na_2CO_3$ and extracted with ethyl acetate (3 x 500 ml). The combined ethyl acetate extracts were washed with water, brine, dried ($MgSO_4$), filtered and concentrated in vacuo leaving dark brown-black oil. Purification of the crude product on a silica gel column (elution with $CH_2Cl_2$, followed by elution with 90% $CH_2Cl_2$-10% $CH_3OH$) gave a total of 7.2 grams (72.8%) of brown crystalline solid; m.p. 62-67° C. MS: $M^+$ = 215. IR(KBr) 5.9 (s) c=O. NMR($CDCl_3$)delta: 3.6 (s, 2H), 6.7-7.4 (m, 7H).

The following 1-(heteroaryl)indoles are prepared according to the above procedure from the products of Preparation A.

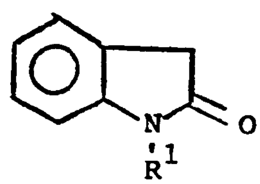

| $R^1$ | MS: $M^+$ |
|---|---|
| 2-thienyl | 215 |
| 3-furyl[(a)] | 199 (100) |
| 2-thiazolyl[(b)] | 216 (78) |

(a) $^1$H-NMR($CDCl_3$)delta: 7.8-6.6 (m, 7H), 3.55 (s, 2H).

(b) TLC (3% $CH_3OH/CH_2Cl_2$): $R_f$ = 0.35
$^1$H-NMR($CDCl_3$)delta: 7.0-7.6 (m, 6H), 3.6 (s, 2H).

PREPARATION C

Starting with the appropriate substituted indole and $R^1$Br reactants, the following compounds are prepared according to the procedures of Preparation A.

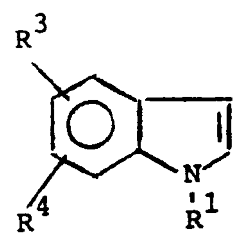

| $R^3$ | $R^4$ | $R^1$ |
|---|---|---|
| 4-F | H | 3-thienyl |
| 5-F | H | 3-thienyl |
| 6-F | H | 3-thienyl |
| H | 7-F | 3-thienyl |
| 4-Cl | H | 3-thienyl |
| 5-Cl | H | 3-thienyl |
| 6-Cl | H | 3-thienyl |
| H | 7-Cl | 3-thienyl |
| 6-$CF_3$ | H | 3-thienyl |
| 5-$CH_3$ | H | 3-thienyl |
| H | 7-$C_2H_5$ | 3-thienyl |
| 6-Br | H | 3-thienyl |
| 4-$OCH_3$ | H | 3-thienyl |
| 4-$SCH_3$ | H | 3-thienyl |
| 5-$NO_2$ | H | 3-thienyl |
| 4-$OCH_3$ | 6-$OCH_3$ | 3-thienyl |
| 4-Cl | 5-$NO_2$ | 3-thienyl |
| 5-$CH_3$ | 7-Cl | 3-thienyl |
| 5-Cl | 6-Cl | 3-thienyl |
| 5-F | 6-F | 3-thienyl |
| 4-$CH_3$ | 7-$CH_3$ | 3-thienyl |
| 5-$OCH_3$ | 6-Cl | 3-thienyl |

| $R^3$ | $R^4$ | $R^1$ |
|---|---|---|
| 5-$OCH_3$ | 6-F | 3-thienyl |
| H | 7-i-$C_4H_9$ | 3-thienyl |
| 5-F | H | 2-thienyl |
| 6-F | H | 2-thienyl |
| 5-Cl | H | 2-thienyl |
| 5-$CF_3$ | H | 2-thienyl |
| 5-$CH_3$ | H | 2-thienyl |
| 6-Br | H | 2-thienyl |
| 4-$SCH_3$ | H | 2-thienyl |
| 4-$OCH_3$ | 6-$OCH_3$ | 2-thienyl |
| 5-$CH_3$ | 7-Cl | 2-thienyl |
| 5-F | 6-F | 2-thienyl |
| 5-$OCH_3$ | 6-F | 2-thienyl |
| 5-$CF_3$ | H | 2-thienyl |
| 5-F | H | 3-furyl |
| 6-Cl | H | 3-furyl |
| 4-$SCH_3$ | H | 3-furyl |
| 6-$CF_3$ | H | 3-furyl |
| 6-Br | H | 3-furyl |
| 4-$SCH_3$ | H | 3-furyl |
| 6-$NO_2$ | H | 3-furyl |
| 4-Cl | 6-Cl | 3-furyl |
| 7-$CH_3$ | H | 3-furyl |
| H | 7-i-$C_4H_9$ | 3-furyl |
| 5-Br | 7-$CH_3$ | 3-furyl |
| 5-$CH_3$ | 6-$CH_3$ | 3-furyl |
| 5-$OCH_3$ | 6-$OCH_3$ | 3-furyl |
| 5-$OCH_3$ | 6-Cl | 3-furyl |
| 5-F | H | 2-furyl |
| 5-Cl | H | 2-furyl |
| 6-$CF_3$ | H | 2-furyl |
| 4-$SCH_3$ | H | 2-furyl |
| 5-$NO_2$ | H | 2-furyl |

| $R^3$ | $R^4$ | $R^1$ |
|---|---|---|
| 5-$OCH_3$ | 6-Cl | 2-furyl |
| 5-$OCH_3$ | 6-$OC_2H_5$ | 2-furyl |
| 5-$CH_3$ | 7-Cl | 2-furyl |
| 5-Cl | 7-Cl | 2-furyl |
| 6-i-$C_3H_7$ | H | 2-furyl |
| 4-$NO_2$ | 7-Cl | 2-furyl |
| 5-$OCH_3$ | 6-F | 2-furyl |
| 5-Br | 7-$CH_3$ | 2-furyl |
| 5-F | H | 2-thiazolyl |
| 6-F | H | 2-thiazolyl |
| 5-Cl | H | 2-thiazolyl |
| 5-Br | H | 2-thiazolyl |
| H | 7-$C_2H_5$ | 2-thiazolyl |
| 6-$CF_3$ | H | 2-thiazolyl |
| 5-$CH_3$ | H | 2-thiazolyl |
| 6-$NO_2$ | H | 2-thiazolyl |
| 6-i-$C_3H_7$ | H | 2-thiazolyl |
| 4-$OCH_3$ | 7-$OCH_3$ | 2-thiazolyl |
| 5-$OCH_3$ | 6-$OCH_3$ | 2-thiazolyl |
| 5-$CH_3$ | 6-$CH_3$ | 2-thiazolyl |
| 5-$OCH_3$ | 6-F | 2-thiazolyl |
| 4-$SCH_3$ | H | 2-thiazolyl |
| 5-$CH_3$ | 6-Cl | 2-thiazolyl |
| 4-$NO_2$ | 7-Cl | 2-thiazolyl |
| 5-Cl | 7-Cl | 2-thiazolyl |

PREPARATION D

The compounds of Preparation C are converted to the corresponding oxindoles having the formula shown below by means of the procedure of Preparation B:

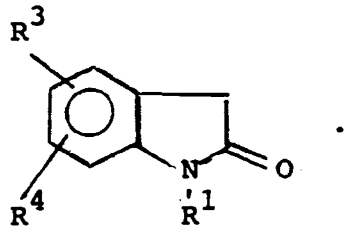

## Claims

**Claims for the following Contracting States : AT, BE, CH, DE, DK, FR, GB, IT, LI, LU, NL, SE**

1. A compound of the formula (I)

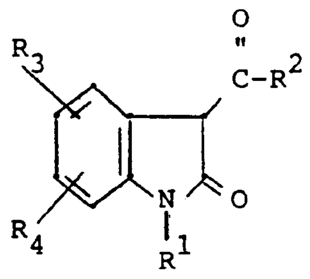

(I)

or a pharmaceutically acceptable base salt or prodrug thereof, wherein
$R^1$ is thienyl, furyl or 2-thiazolyl;
$R^2$ is thienyl, furyl or

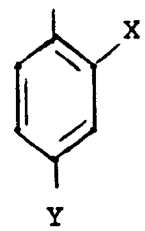

wherein X is hydrogen, fluoro, chloro, alkyl having 1 to 4 carbon atoms, alkoxy having 1 to 4 carbon atoms, alkylthio having 1 to 4 carbon atoms, nitro or trifluoromethyl;
Y is hydrogen, fluoro or chloro;
and each of $R^3$ and $R^4$, which may be alike or different, is hydrogen, fluoro, chloro, bromo, alkyl having 1 to 4 carbon atoms, alkoxy having 1 to 4 carbon atoms, alkylthio having 1 to 4 carbon atoms, nitro or trifluoromethyl.

2. A compound according to claim 1, wherein $R^1$ is thienyl.

3. A compound according to claim 1 or claim 2 wherein $R^1$ is 2-thienyl or 3-thienyl and $R^2$ is

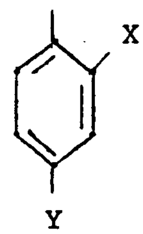

wherein X and Y are defined as in claim 1.

4. A compound according to claim 3, wherein X is H, F, Cl, -S($C_1$-$C_4$ alkyl) or $CF_3$; Y is H, F or Cl; and each of $R^3$ and $R^4$ is hydrogen.

5. A compound according to claim 1, wherein $R^1$ is furyl.

6. A compound according to claim 5, wherein $R^1$ is 3-furyl and $R^2$ is

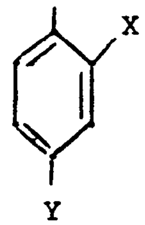

wherein X and Y are defined as in claim 1.

7. A compound according any one of claims 3 and 6, wherein each of X and Y is H, F or Cl; and each of $R^3$ and $R^4$ is hydrogen.

8. A compound according to any one of claims 1 to 7, wherein said compound is a prodrug of said compound of the formula (I), said prodrug having the formula

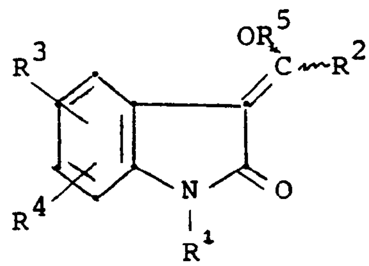

(II)

wherein each of $R^1$, $R^2$, $R^3$ and $R^4$ is as defined in claim 1, and $R^5$ is alkanoyl, cycloalkylcarbonyl, phenylalkanoyl, chlorobenzoyl, methoxybenzyl, phenyl, thenoyl, omegaalkoxycarbonylalkanoyl, alkoxycarbonyl, phenoxycarbonyl, 1-alkoxyalkyl, 1-alkoxycarbonyloxyalkyl, alkyl, alkylsulfonyl, methylphenylsulfonyl or dialkylphosphonate.

9. A pharmaceutical composition comprising a compound of the formula (I), or a pharmaceutically acceptable base salt or prodrug thereof as claimed in any one of claims 1 to 8, together with a pharmaceutically acceptable diluent or carrier.

10. A compound of the formula (I), or a pharmaceutically acceptable base salt, prodrug, or composition thereof as claimed in any one of claims 1 to 8 and 9 respectively, for use as a medicament.

11. The use of a compound of the formula (I), or of a pharmaceutically acceptable base salt, prodrug or composition thereof as claimed in any one of claims 1 to 8 and 9 respectively, for the manufacture of a medicament for the treatment of an inflammatory disease in a mammal, in particular for the treatment of rheumatoid arthritis.

**Claims for the following Contracting States : ES, GR**

1. A process for preparing a compound of the formula (I)

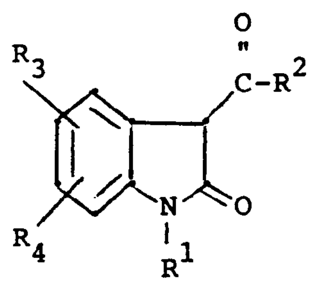

(I)

or a pharmaceutically acceptable base salt thereof, wherein

$R^1$ is thienyl, furyl or 2-thiazolyl;

$R^2$ is thienyl, furyl or

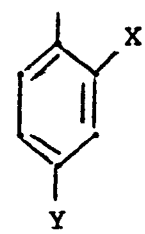

wherein X is hydrogen, fluoro, chloro, alkyl having 1 to 4 carbon atoms, alkoxy having 1 to 4 carbon atoms, alkylthio having 1 to 4 carbon atoms, nitro or trifluoromethyl;

Y is hydrogen, fluoro or chloro;

and each of $R^3$ and $R^4$, which may be alike or different, is hydrogen, fluoro, chloro, bromo, alkyl having 1 to 4 carbon atoms, alkoxy having 1 to 4 carbon atoms, alkylthio having 1 to 4 carbon atoms, nitro or trifluoromethyl;

said process comprising reacting a compound of the formula

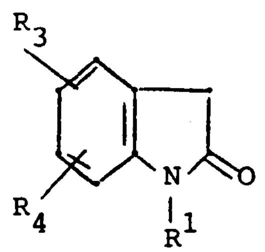

(c)

wherein $R^1$, $R^3$ and $R^4$ are as defined above, with a compound of the formula $R^2COCl$, wherein $R^2$ is as defined above;

and, optionally, converting said compound of formula I into a pharmaceutically acceptable base salt thereof by reacting it with a pharmaceutically acceptable salt producing base.

2. A process according to claim 1, wherein a compound of said formula I, wherein $R^1$ is thienyl and $R^3$ and $R^4$ are as defined in claim 1, is prepared by reacting a compound of said formula (c), wherein $R^1$ is thienyl and $R^3$ and $R^4$ are as defined in claim 1, with said compound of the formula $R^2COCl$, wherein $R^2$ is defined as in claim 1.

3. A process according to claim 1 or 2, wherein a compound of said formula I, wherein $R^1$ is 2-thienyl or 3-thienyl and $R^3$ and $R^4$ are as defined in claim 1, and $R^2$ is

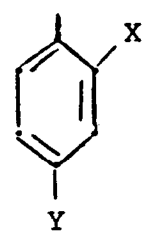

wherein X and Y are as defined in claim 1, is prepared by reacting a compound of said formula (c), wherein $R^1$ is 2-thienyl or 3-thienyl and $R^3$ and $R^4$ are as defined in claim 1, with said compound of the formula $R^2COCl$, wherein $R^2$ is as defined above.

4. A process according to claim 1 or claim 3, wherein a compound of said formula (I), wherein $R^2$ is as defined

in claim 3, X is H, F, Cl, $S(C_1-C_4)$ alkyl or $CF_3$, Y is H, F or Cl, and each of $R^3$ and $R^4$ is hydrogen, is prepared by reacting a compound of said formula (c), wherein each of $R^3$ and $R^4$ is hydrogen, with said compound of the formula $R^2COCl$, wherein $R^2$ is as defined in claim 3, X is H, F, Cl, $S(C_1-C_4)$alkyl or $CF_3$, and Y is H, F or Cl.

5. A process according to claim 1, wherein a compound of said formula I, wherein $R^1$ is furyl, is prepared by reacting a compound of said formula (c), wherein $R^1$ is furyl, with a compound of said formula $R^2COCl$, wherein $R^2$ is as defined in claim 1.

6. A process according to claim 1 or claim 5, wherein a compound of said formula (I), wherein $R^1$ is 3-furyl, $R^3$ and $R^4$ are as defined in claim 1, and $R^2$ is

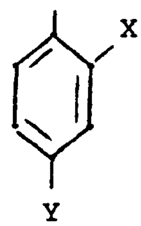

,

wherein X and Y are as defined in claim 1, is prepared by reacting a compound of said formula (c), wherein $R^1$ is 3-furyl and $R^3$ and $R^4$ are as defined in claim 1, with a compound of said formula $R^2COCl$, wherein $R^2$ is as defined above.

7. A process according to claim 6,
wherein a compound of said formula (I), wherein $R^1$ and $R^2$ are as defined in claim 6, each of X and Y is H, F or Cl and each of $R^3$ and $R^4$ is H, is prepared by reacting a compound of said formula (c), wherein $R^1$ is as defined in claim 6, and each of $R^3$ and $R^4$ is H, with a compound of said formula $R^2COCl$, wherein $R^2$ is as defined in claim 6 and each of X and Y is H, F or Cl.

8. A process for preparing a compound of the formula

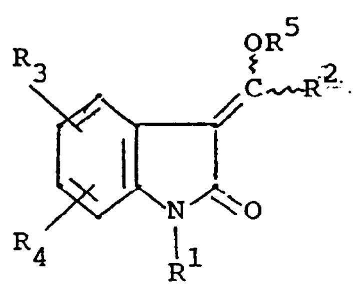

(II)

wherein each of $R^1$, $R^2$, $R^3$ and $R^4$ is as defined in claim 1 and $R^5$ is alkanoyl, cycloalkylcarbonyl, phenylalkanoyl, chlorobenzoyl, methoxybenzyl, phenyl, thenoyl, omegaalkoxycarbonylalkanoyl, alkoxycarbonyl, phenoxycarbonyl, 1-alkoxyalkyl, 1-alkoxycarbonyloxyalkyl, alkyl, alkylsulfonyl, methylphenylsulfonyl or dialkylphosphonate;

said process comprising reacting a compound of said formula (I), as defined in claim 1, with an acid chloride, chloroformate, oxonium salt or alkylating agent.

9. A process according to claim 8, wherein said compound of the formula (I) is reacted with an alpha-chloroalkylcarbonate, sodium iodide and potassium carbonate.

10. A process according to claim 1 wherein said compound of the formula (c) is obtained by reacting a compound of the formula

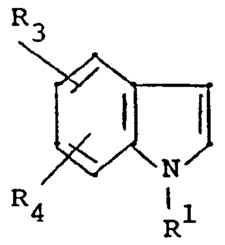

(b)

wherein $R^1$, $R^3$ and $R^4$ are as defined in claim 1, with N-chlorosuccinimide followed by treatment with phosphoric acid.

## Patentansprüche

**Patentansprüche für folgende Vertragsstaaten : AT, BE, CH, DE, DK, FR, GB, IT, LI, LU, NL, SE**

1. Verbindung der Formel (I)

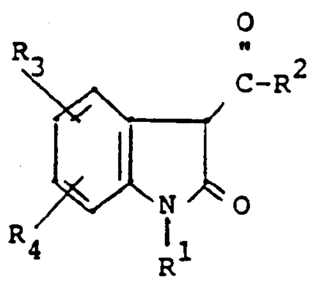

(I)

oder ein pharmazeutisch verträgliches Basesalz oder Prodrug davon, wobei
$R^1$ Thienyl, Furyl oder 2-Thiazolyl bedeutet;
$R^2$ Thienyl, Furyl oder

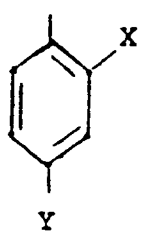

bedeutet, worin X ein Wasserstoffatom, Fluor, Chlor, Alkyl mit 1 bis 4 Kohlenstoffatomen, Alkoxy mit 1 bis 4 Kohlenstoffatomen, Alkylthio mit 1 bis 4 Kohlenstoffatomen, Nitro oder Trifluormethyl bedeutet;
Y ein Wasserstoffatom, Fluor oder Chlor darstellt und jeder der Reste $R^3$ und $R^4$, der gleich oder verschieden sein kann, ein Wasserstoffatom, Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Nitro oder Trifluormethyl bedeutet.

2. Verbindung nach Anspruch 1, wobei $R^1$ Thienyl bedeutet.

3. Verbindung nach Anspruch 1 oder 2, wobei $R^1$ 2-Thienyl oder 3-Thienyl bedeutet und $R^2$

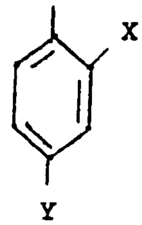

bedeutet, worin X und Y wie in Anspruch 1 definiert sind.

4. Verbindung nach Anspruch 3, wobei X H, F, Cl, -S($C_1$-$C_4$-Alkyl) oder $CF_3$ bedeutet; Y H, F oder Cl darstellt; und jeder der Reste $R^3$ und $R^4$ ein Wasserstoffatom bedeutet.

5. Verbindung nach Anspruch 1, wobei $R^1$ Furyl darstellt.

6. Verbindung nach Anspruch 5, wobei $R^1$ 3-Furyl darstellt und $R^2$

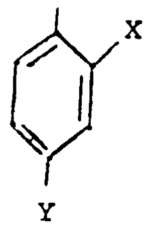

bedeutet, worin X und Y wie in Anspruch 1 definiert sind.

7. Verbindung nach einem der Ansprüche 3 und 6, wobei jeder der Reste X und Y H, F oder Cl bedeutet und jeder der Reste $R^3$ und $R^4$ ein Wasserstoffatom bedeutet.

8. Verbindung nach einem der Ansprüche 1 bis 7, wobei die Verbindung ein Prodrug der Verbindung der Formel (I) darstellt, wobei das Prodrug die Formel

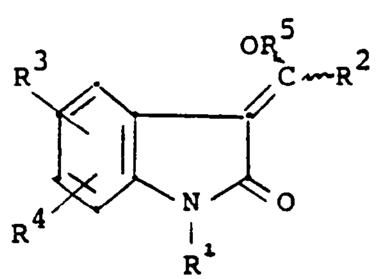

(II)

aufweist, worin jeder der Reste $R^1$, $R^2$, $R^3$ und $R^4$ wie in Anspruch 1 definiert ist und $R^5$ Alkanoyl, Cycloalkylcarbonyl, Phenylalkanoyl, Chlorbenzoyl, Methoxybenzyl, Phenyl, Thenoyl, ω-Alkoxycarbonylalkanoyl, Alkoxycarbonyl, Phenoxycarbonyl, 1-Alkoxyalkyl, 1-Alkoxycarbonyloxyalkyl, Alkyl, Alkylsulfonyl, Methylphenylsulfonyl oder Dialkylphosphonat bedeutet.

9. Arzneimittel, umfassend eine Verbindung der Formel (I) oder ein pharmazeutisch verträgliches Basesalz oder Prodrug davon gemäß einem der Ansprüche 1 bis 8 zusammen mit einem pharmazeutischen Verdünnungs- oder Trägermittel.

10. Verbindung der Formel (I) oder ein pharmazeutisch verträgliches Basesalz, Prodrug oder Mittel davon gemäß einem der Ansprüche 1 bis 8 bzw. 9 zur Verwendung als Arzneimittel.

11. Verwendung einer Verbindung der Formel (I) oder eines pharmazeutisch verträgliches Basesalzes, Prodrugs oder Mittels davon gemäß einem der Ansprüche 1 bis 8 bzw. 9 zur Herstellung eines Arzneimittels zur Behandlung einer entzündlichen Erkrankung bei einem Säuger, insbesondere zur Behandlung rheumatischer Arthritis.

**Patentansprüche für folgende Vertragsstaaten : ES, GR**

1. Verfahren zur Herstellung einer Verbindung der Formel (I)

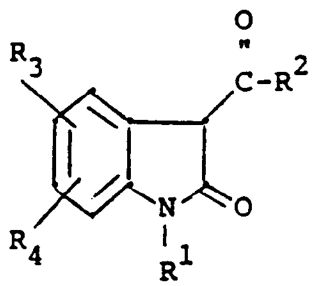

(I)

oder eines pharmazeutisch verträglichen Basesalzes davon, wobei
$R^1$ Thienyl, Furyl oder 2-Thiazolyl bedeutet;
$R^2$ Thienyl, Furyl oder

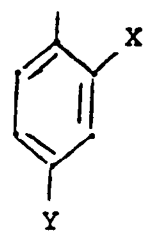

bedeutet, worin X ein Wasserstoffatom, Fluor, Chlor, Alkyl mit 1 bis 4 Kohlenstoffatomen, Alkoxy mit 1 bis 4 Kohlenstoffatomen, Alkylthio mit 1 bis 4 Kohlenstoffatomen, Nitro oder Trifluormethyl bedeutet;
Y ein Wasserstoffatom, Fluor oder Chlor darstellt
und jeder der Reste $R^3$ und $R^4$, der gleich oder verschieden sein kann, ein Wasserstoffatom, Fluor, Chlor, Brom, Alkyl mit 1 bis 4 Kohlenstoffatomen, Alkoxy mit 1 bis 4 Kohlenstoff-atomen, Alkylthio mit 1 bis 4 Kohlenstoffatomen, Nitro oder Trifluormethyl bedeutet;
wobei das Verfahren umfaßt: Umsetzung einer Verbindung der Formel

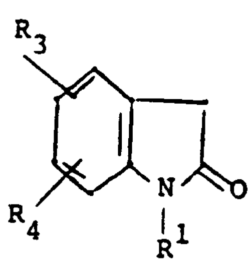

(c)

worin $R^1$, $R^3$ und $R^4$ wie vorstehend definiert sind mit einer Verbindung der Formel $R^2COCl$, worin $R^2$ wie vorstehend definiert ist;
und gegebenenfalls Umwandeln der Verbindung von Formel (I) in ein pharmazeutisch verträgliches Basesalz davon durch Umsetzen mit einer Base, die ein pharmazeutisch verträgliches Basesalz erzeugt.

2. Verfahren nach Anspruch 1, wobei eine Verbindung der Formel I, worin $R^1$ Thienyl bedeutet und $R^3$ und $R^4$ wie in Anspruch 1 definiert sind, durch Umsetzen einer Verbindung der Formel (c) hergestellt wird, worin $R^1$ Thienyl bedeutet und $R^3$ und $R^4$ wie in Anspruch 1 definiert sind, mit einer Verbindung der Formel $R^2COCl$, worin $R^2$ wie in Anspruch 1 definiert ist.

3. Verfahren nach Anspruch 1 oder 2, wobei eine Verbindung der Formel I, in der $R^1$ 2-Thienyl oder 3-Thienyl bedeutet und $R^3$ und $R^4$ wie in Anspruch 1 definiert sind, und $R^2$

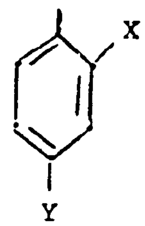

darstellt, worin X und Y wie in Anspruch 1 definiert sind, durch Umsetzen einer Verbindung der Formel (c), worin $R^1$ 2-Thienyl oder 3-Thienyl bedeutet und $R^3$ und $R^4$ wie in Anspruch 1 definiert sind, mit einer Verbindung der Formel $R^2COCl$, worin $R^2$ wie vorstehend definiert ist, hergestellt wird.

4. Verfahren nach Anspruch 1 oder Anspruch 3, wobei eine Verbindung der Formel (I), worin $R^2$ wie in Anspruch 3 definiert ist, X H, F, Cl, $-S(C_1-C_4\text{-Alkyl})$ oder $CF_3$ bedeutet; Y H, F oder Cl darstellt; und jeder der Reste $R^3$ und $R^4$ ein Wasserstoffatom bedeutet, durch Umsetzen einer Verbindung der Formel (c), wobei jeder der Reste $R^3$ und $R^4$ ein Wasserstoffatom bedeutet, mit einer Verbindung der Formel $R^2COCl$, worin $R^2$ wie in Anspruch 3 definiert ist, X H, F, Cl, $-S(C_1-C_4\text{-Alkyl})$ oder $CF_3$ bedeutet; Y H, F oder Cl darstellt, hergestellt wird.

5. Verfahren nach Anspruch 1, wobei eine Verbindung der Formel (I), in der $R^1$ Furyl bedeutet, durch Umsetzen einer Verbindung der Formel (c), worin $R^1$ Furyl bedeutet, mit einer Verbindung der Formel $R^2COCl$, worin $R^2$ wie in Anspruch 1 definiert ist, hergestellt wird.

6. Verfahren nach Anspruch 1 oder Anspruch 5, wobei eine Verbindung der Formel (I), worin $R^1$ 3-Furyl bedeutet, die Reste $R^3$ und $R^4$ wie in Anspruch 1 definiert sind, und $R^2$

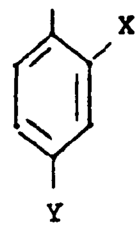

bedeutet, worin X und Y wie in Anspruch 1 definiert sind, durch Umsetzen einer Verbindung der Formel (c), worin $R^1$ 3-Furyl bedeutet und $R^3$ und $R^4$ wie in Anspruch 1 definiert sind, mit einer Verbindung der Formel $R^2COCl$, worin $R^2$ wie vorstehend definiert ist, hergestellt wird.

7. Verfahren nach Anspruch 6, wobei eine Verbindung der Formel (I), worin $R^1$ und $R^2$ wie in Anspruch 6 definiert sind, jeder der Reste X und Y H, F oder Cl bedeutet, jeder der Reste $R^3$ und $R^4$ H bedeutet, durch Umsetzen einer Verbindung der Formel (c), worin $R^1$ wie in Anspruch 6 definiert ist und jeder der Reste $R^3$ und $R^4$ H bedeutet, mit einer Verbindung der Formel $R^2COCl$, worin $R^2$ wie in Anspruch 6 definiert ist und jeder der Reste X und Y H, F oder Cl bedeutet, hergestellt wird.

8. Verfahren zur Herstellung einer Verbindung der Formel

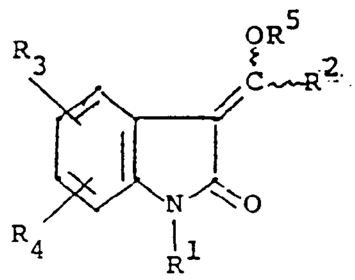

(II)

worin jeder der Reste $R^1$, $R^2$, $R^3$ und $R^4$ wie in Anspruch 1 definiert ist und $R^5$ Alkanoyl, Cycloalkylcarbonyl, Phenylalkanoyl, Chlorbenzoyl, Methoxybenzyl, Phenyl, Thenoyl, ω-Alkoxycarbonylalkanoyl, Alkoxycarbonyl, Phenoxycarbonyl, 1-Alkoxyalkyl, 1-Alkoxycarbonyloxyalkyl, Alkyl, Alkylsulfonyl, Methylphenylsulfonyl oder Dialkylphosphonat bedeutet, wobei das Verfahren umfaßt;

Umsetzen einer Verbindung der Formel (I) gemäß Anspruch 1 mit einem Säurechlorid, Chlorameisensäureester, Oxoniumsalz oder einem Alkylierungsmittel.

**9.** Verfahren nach Anspruch 8, wobei die Verbindung der Formel (I) umgesetzt wird mit einem α-Kohlensäurealkylester, Natriumjodid und Kaliumcarbonat.

**10.** Verfahren nach Anspruch 1, wobei die Verbindung der Formel (c) erhalten wird durch Umsetzen einer Verbindung der Formel

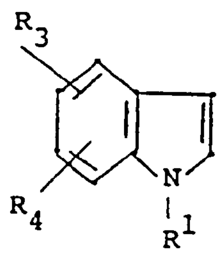

(b)

worin $R^1$, $R^3$ und $R^4$ wie in Anspruch 1 definiert sind, mit N-Chlorsuccinimid, gefolgt von einer Behandlung mit Phosphorsäure.

## Revendications

**Revendications pour les Etats contractants suivants : AT, BE, CH, DE, DK, FR, GB, IT, LI, LU, NL, SE**

**1.** Composé de formule (I)

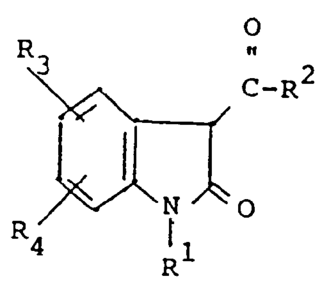

(I)

ou un de ses sels de base pharmaceutiquement acceptables ou précurseurs médicamenteux, formule dans laquelle

$R^1$ représente un groupe thiényle, furyle ou 2-thiazolyle ;

$R^2$ représente un groupe thiényle, furyle ou

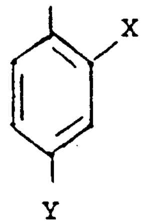

dans lequel X représente l'hydrogène, un groupe fluoro, chloro, alkyle ayant 1 à 4 atomes de carbone, alkoxy ayant 1 à 4 atomes de carbone, alkylthio ayant 1 à 4 atomes de carbone, nitro ou trifluorométhyle ;

Y représente l'hydrogène, un groupe fluoro ou chloro ;

et chacun des groupes $R^3$ et $R^4$, qui peuvent être identiques ou différents, représente l'hydrogène, un groupe fluoro, chloro, bromo, alkyle ayant 1 à 4 atomes de carbone, alkoxy ayant 1 à 4 atomes de carbone, alkylthio ayant 1 à 4 atomes de carbone, nitro ou trifluorométhyle.

2. Composé suivant la revendication 1, dans lequel $R^1$ représente un groupe thiényle.

3. Composé suivant la revendication 1 ou la revendication 2, dans lequel $R^1$ représente un groupe 2-thiényle ou 3-thiényle et $R^2$ représente un groupe

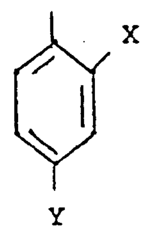

dans lequel X et Y répondent aux définitions suivant la revendication 1.

4. Composé suivant la revendication 3, dans lequel X représente H, F, Cl, un groupe -S(alkyle en $C_1$ à $C_4$) ou $CF_3$ ; Y représente H, F ou Cl ; et chacun des groupes $R^3$ et $R^4$ représente l'hydrogène.

5. Composé suivant la revendication 1, dans lequel $R^1$ représente un groupe furyle.

6. Composé suivant la revendication 5, dans lequel $R^1$ représente un groupe 3-furyle et $R^2$ représente un groupe

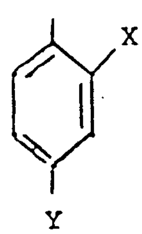

dans lequel X et Y répondent aux définitions suivant la revendication 1.

7. Composé suivant l'une quelconque des revendications 3 à 6, dans lequel chacun des groupes X et Y représente H, F ou Cl ; et chacun des groupes $R^3$ et $R^4$ représente l'hydrogène.

8. Composé suivant l'une quelconque des revendications 1 à 7, qui est un précurseur médicamenteux du composé de formule (I), ledit précurseur médicamenteux répondant à la formule

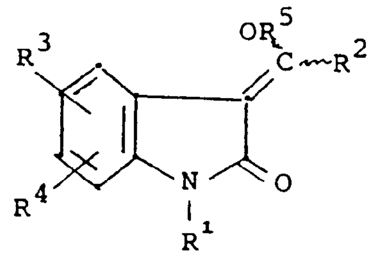

(II)

dans laquelle chacun des groupes $R^1$, $R^2$, $R^3$ et $R^4$ répond à la définition suivant la revendication 1, et $R^5$ représente un groupe alcanoyle, cycloalkylcarbonyle, phénylalcanoyle, chlorobenzoyle, méthoxybenzyle, phényle, thénoyle, oméga-alkoxycarbonylalcanoyle, alkoxycarbonyle, phénoxycarbonyle, 1-alkoxyalkyle, 1-alkoxycarbonyloxyalkyle, alkyle, alkylsulfonyle, méthylphénylsulfonyle ou dialkylphosphonate.

**9.** Composition pharmaceutique comprenant un composé de formule (I), ou un de ses sels de base pharmaceutiquement acceptables ou précurseurs médicamenteux suivant l'une quelconque des revendications 1 à 8, en association avec un diluant ou support pharmaceutiquement acceptable.

**10.** Composé de formule (I), ou un de ses sels de base pharmaceutiquement acceptables ou précurseurs médicamenteux ou bien une de ses compositions suivant l'une quelconque des revendications 1 à 8 et 9, respectivement, destiné à être utilisé comme médicament.

**11.** Utilisation d'un composé de formule (I), ou un de ses sels de base pharmaceutiquement acceptables ou précurseurs médicamenteux ou bien une de ses compositions suivant l'une quelconque des revendications 1 à 8 et 9, respectivement, pour la production d'un médicament destiné au traitement d'une maladie inflammatoire chez un mammifère, en particulier au traitement de l'arthrite rhumatoïde.

**Revendications pour les Etats contractants suivants : ES, GR**

**1.** Procédé de préparation d'un composé de formule (I)

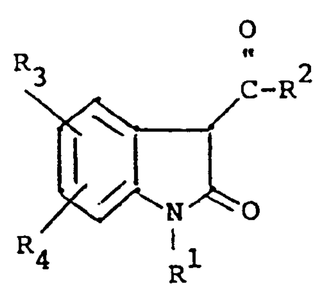

(I)

ou d'un des sels de base pharmaceutiquement acceptables, formule dans laquelle

$R^1$ représente un groupe thiényle, furyle ou 2-thiazolyle ;

$R^2$ représente un groupe thiényle, furyle ou

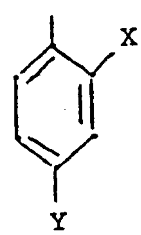

dans lequel X représente l'hydrogène, un groupe fluoro, chloro, alkyle ayant 1 à 4 atomes de carbone, alkoxy ayant 1 à 4 atomes de carbone, alkylthio ayant 1 à 4 atomes de carbone, nitro ou trifluorométhyle ;

Y représente l'hydrogène, un groupe fluoro ou chloro ;

et chacun des groupes $R^3$ et $R^4$, qui peuvent être identiques ou différents, représente l'hydrogène, un groupe fluoro, chloro, bromo, alkyle ayant 1 à 4 atomes de carbone, alkoxy ayant 1 à 4 atomes de carbone, alkylthio ayant 1 à 4 atomes de carbone, nitro ou trifluorométhyle ;

ledit procédé comprenant la réaction d'un composé de formule

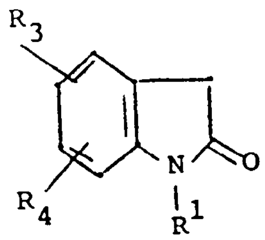

(c)

dans laquelle $R^1$, $R^3$ et $R^4$ répondent aux définitions précitées, avec un composé de formule $R^2COCl$, dans laquelle $R^2$ répond à la définition précitée ;

et, facultativement, la transformation dudit composé de formule I en un de ses sels de base pharmaceutiquement acceptables par réaction de ce composé avec une base donnant un sel pharmaceutiquement acceptable.

**2.** Procédé suivant la revendication 1, dans lequel un composé de formule I, dans laquelle $R^1$ représente un groupe thiényle et $R^3$ et $R^4$ répondent aux définitions suivant la revendication 1, est préparé par réaction d'un composé de formule (c), dans laquelle $R^1$ représente un groupe thiényle et $R^3$ et $R^4$ répondent aux définitions suivant la revendication 1, avec le composé de formule $R^2COCl$, dans laquelle $R^2$ répond à la définition suivant la revendication 1.

**3.** Procédé suivant la revendication 1 ou 2, dans lequel un composé de formule I, dans laquelle $R^1$ représente un groupe 2-thiényle ou 3-thiényle et $R^3$ et $R^4$ répondent aux définitions suivant la revendication 1, et $R^2$ représente un groupe

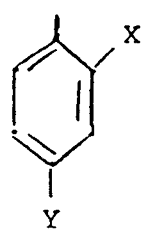

dans lequel X et Y répondent aux définitions suivant la revendication 1, est préparé par réaction d'un composé de formule (c), dans laquelle $R^1$ représente un groupe 2-thiényle ou 3-thiényle et $R^3$ et $R^4$ répondent aux définitions suivant la revendication 1, avec le composé de formule $R^2COCl$, dans laquelle $R^2$ répond à la définition précitée.

**4.** Procédé suivant la revendication 1 ou la revendication 3, dans lequel un composé de formule (I), dans laquelle $R^2$ répond à la définition suivant la revendication 3, X représente H, F, Cl, un groupe -S(alkyle en $C_1$ à $C_4$) ou $CF_3$, Y représente H, F ou Cl, et chacun des groupes $R^3$ et $R^4$ représente l'hydrogène, est préparé par réaction d'un composé de formule (c), dans laquelle chacun des groupes $R^3$ et $R^4$ représente l'hydrogène, avec le composé de formule $R^2COCl$, dans laquelle $R^2$ répond à la définition suivant la revendication 3, X représente H, F, Cl, un groupe -S(alkyle en $C_1$ à $C_4$) ou $CF_3$, Y représente H, F ou Cl.

**5.** Procédé suivant la revendication 1, dans lequel un composé de formule I, dans laquelle $R^1$ représente un groupe furyle, est préparé par réaction d'un composé de formule (c), dans laquelle $R^1$ représente un groupe furyle, avec un composé de formule $R^2COCl$, dans laquelle $R^2$ répond à la définition suivant la revendication 1.

**6.** Procédé suivant la revendication 1 ou la revendication 5, dans lequel un composé de formule (I), dans laquelle $R^1$ représente un groupe 3-furyle, $R^3$ et $R^4$ répondent aux définitions suivant la revendication 1 et $R^2$ représente un groupe

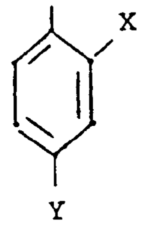

dans lequel X et Y répondent aux définitions suivant la revendication 1, est préparé par réaction d'un composé de formule (c), dans laquelle $R^1$ représente un groupe 3-furyle et $R^3$ et $R^4$ répondent aux définitions suivant la revendication 1, avec un composé de formule $R^2COCl$, dans laquelle $R^2$ répond à la définition précitée.

7. Procédé suivant la revendication 6, dans lequel un composé de formule (I), dans laquelle $R^1$ et $R^2$ répondent aux définitions suivant la revendication 6, chacun des groupes X et Y représente H, F ou Cl et chacun des groupes $R^3$ et $R^4$ représente H, est préparé par réaction d'un composé de formule (c), dans laquelle $R^1$ répond à la définition suivant la revendication 6, et chacun des groupes $R^3$ et $R^4$ représente H, avec un composé de formule $R^2COCl$, dans laquelle $R^2$ répond à la définition suivant la revendication 6 et chacun des groupes X et Y représente H, F ou Cl.

8. Procédé de préparation d'un composé de formule

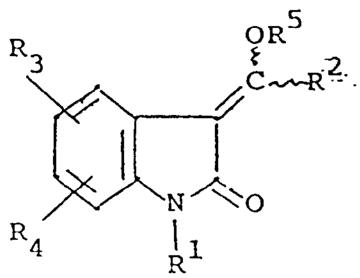

(II)

dans laquelle chacun des groupes $R^1$, $R^2$, $R^3$ et $R^4$ répond à la définition suivant la revendication 1, et $R^5$ représente un groupe alcanoyle, cycloalkylcarbonyle, phénylalcanoyle, chlorobenzoyle, méthoxybenzyle, phényle, thénoyle, oméga-alkoxycarbonylalcanoyle, alkoxycarbonyle, phénoxycarbonyle, 1-alkoxyalkyle, 1-alkoxycarbonyloxyalkyle, alkyle, alkylsulfonyle, méthylphénylsulfonyle ou dialkylphosphonate ;

ledit procédé comprenant la réaction d'un composé de formule (I), définie suivant la revendication 1, avec un chlorure d'acide, un chloroformiate, un sel d'oxonium ou un agent d'alkylation.

9. Procédé suivant la revendication 8, dans lequel le composé de formule (I) est amené à réagir avec un alpha-chloralkylcarbonate, l'iodure de sodium et le carbonate de potassium.

10. Procédé suivant la revendication 1, dans lequel le composé de formule (c) est obtenu par réaction d'un composé de formule

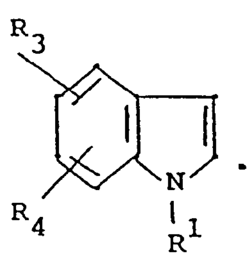

(b)

dans laquelle $R^1$, $R^3$ et $R^4$ répondent aux définitions suivant la revendication 1, avec le N-chlorosuccinimide, puis par traitement avec l'acide phosphorique.